Europäisches Patentamt

European Patent Office

Office européen des brevets

(11) Publication number: **0 246 908**
**A2**

# (12) EUROPEAN PATENT APPLICATION

(21) Application number: **87304556.1**

(22) Date of filing: **21.05.87**

(51) Int. Cl.⁴: **A 61 N 1/37**

(30) Priority: **23.05.86 GB 8612659**

(43) Date of publication of application:
**25.11.87 Bulletin 87/48**

(84) Designated Contracting States:
**AT BE CH DE ES FR GB GR IT LI LU NL SE**

(71) Applicant: **COVENTRY CITY COUNCIL**
**The Council House Earl Street**
**Coventry West Midlands CV1 5RR (GB)**

(72) Inventor: **Economides, Apollo Plato**
**33 Baginton Road**
**Coventry West Midlands CV3 6FZ (GB)**

**Gergely, Stephen**
**91 Maidavale Crescent**
**Coventry West Midlands CV3 5FZ (GB)**

**Walton, Christopher**
**72 Silverdale Close**
**Coventry West Midlands CV2 1PY (GB)**

(74) Representative: **Hallam, Arnold Vincent et al**
**T. Fletcher Wilson & Co. 9 Grosvenor House Grosvenor Road**
**Coventry West Midlands, CV1 3FZ (GB)**

(54) Cardiac pacemaker circuit.

(57) This invention relates to a cardiac pacemaker circuit in which a stimulating electrode is embedded in a patient's atrium or ventricle and a reference electrode positioned outside the heart or on the same lead within the heart. Stimulus pulses are applied across the two electrodes, these replacing the evoked pulses. On termination of the stimulus pulse the amount of charge stored between the two electrodes is monitored and a pulse applied to the stimulating electrode to reduce this charge to a level at which the intrinsic pulses of the evoked heart response can be monitored. The evoked heart response can then be used for diagnosis whilst the pacemaker circuit is in operation. If the stored charge were not reduced then the charge would mask the intrinsic pulses and the pacemaker would have to be switched off before these could be monitored for diagnosis.

FIG.2.

**Description**

"CARDIAC PACEMAKER CIRCUIT"

This invention relates to a cardiac pacemaker circuit.

Intrinsic cardiac pulses may be measured using an electrode placed in or in the vicinity of a heart and an example of such pulses for a healthy patient is shown in Figure IA. For each heartbeat, the pulses comprise a wave labelled P which causes the atria to contract, and a complex wave form labelled QRST associated with the contraction of the ventricles. A pacemaker circuit comprises a pulse generator connected to a stimulating electrode embedded in the patient's atrium or ventricle and a reference electrode positioned outside the heart in a unipolar configuration, or on the same lead within the heart in a bipolar system. The generator applies stimulus pulses across these two electrodes which replaces the evoked pulses. During each stimulus pulse, electrical charge is stored in the interface between the stimulus electrode and the myocardium, and the charge then decays upon termination of the stimulus pulse. An example of the waveform appearing at the stimulus electrode is shown in Figure IB in which each stimulus pulse is denoted by St. As may be seen, the charge which is stored and its subsequent decay masks the evoked heart response. However, it is useful to detect these evoked pulses as they may be used both for diagnosis and for modifying the operation of the pacemaker circuit.

It is accordingly an object of this invention to provide a new pacemaker circuit in which the masking of the evoked heart pulses by the charge stored between the stimulus electrode and the myocardium is reduced or removed.

According to this invention there is provided a cardiac pacemaker circuit including a terminal for connection to a stimulatings electrode, a terminal for connection to a reference electrode, means for applying a stimulus pulse across said stimulating terminal and said reference terminal in response to a timing signal, means for generating said timing signal, and means connected to said stimulating electrode and said reference electrode which are operable, in use, upon termination of the stimulus pulse to remove charge stored between the stimulating electrode and the reference electrode.

By removing the charge which is stored between the stimulating electrode and the tissue, the masking which is caused by such charge is removed.

Said stimulus pulse applying means may comprise a reference voltage source, a resistor and capacitor connected in series between the voltage reference source and the stimulating terminal, and a switch responsive to the timing signal connected between the junction of the resistor and capacitor and the reference terminal.

The charge removing means may be connected to the junction of the resistor and capacitor.

In a preferred embodiment of this invention, said charge removing means is arranged to sample the voltage difference between the stimulating terminal and the reference terminal upon termination of a stimulus pulse and to apply an electrical pulse across the electrodes in accordance with the result of the sampling operation.

The charge removing means may be arranged to form a plurality of sampling operations upon termination of a stimulus pulse, each sampling operation being followed by the application of an electrical pulse.

In an alternative arrangement of this invention, the charge removing means is arranged to measure the quantity of charge supplied to a stimulus pulse and to remove substantially an identical quantity of charge upon termination of the stimulus pulse.

The charge removing means may be arranged to clamp the potential difference between the stimulating electrode and the reference electrode for a preset period after removing said substantially identical quantity of charge.

The pacemaker circuit may include a resistor connected in series with one of the stimulating electrode and the reference electrode, the charge supplied during the stimulus pulse and the charge subsequently removed being measured by integrating the voltage across said resistor with respect to time.

This invention will now be described in more details with reference to the accompanying drawings in which:-

Figures IA and IB show waveforms of the electrical activity appearing in the heart of a healthy patient and a waveform appearing at the stimulating electrode of a pacemaker circuit;

Figure 2 is a block diagram of a preferred embodiment of this invention;

Figures 3 and 4 are detailed circuit diagrams of the embodiments shown in Figure 2;

Figure 5 shows waveforms appearing in the embodiment of Figure 2; and

Figure 6 is a circuit diagram of another embodiment of this invention.

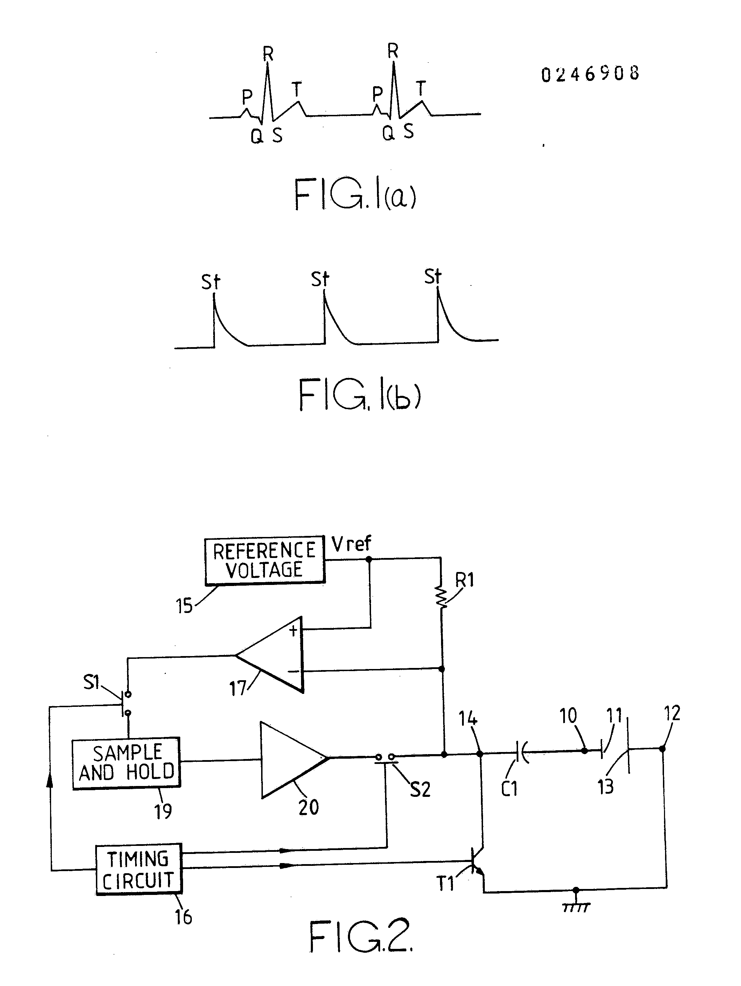

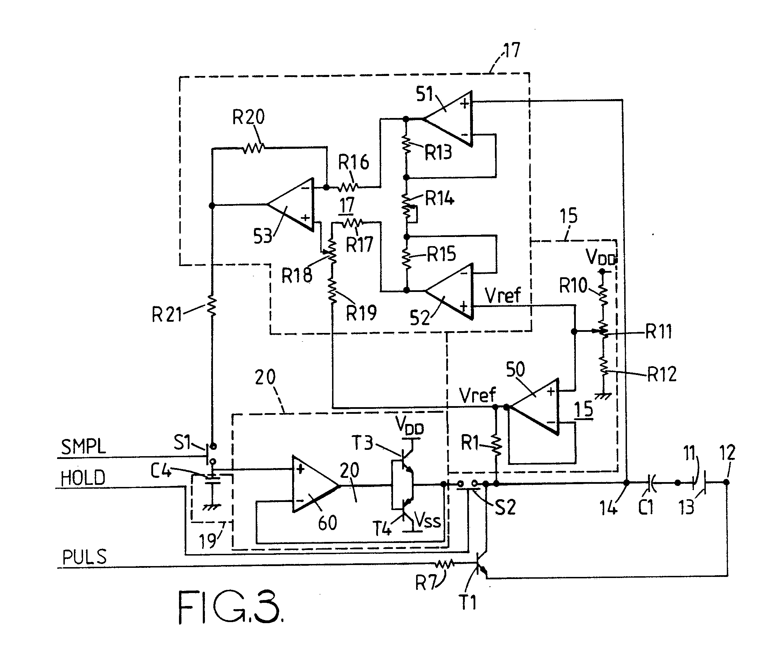

Referring now to Figure 2, there is shown a cardiac pacemaker circuit which has a stimulating terminal 10 connected to a stimulating electrode 11 and a reference terminal 12 connected to a reference electrode 13. The reference terminal 12 is connected to the earth line. The stimulating terminal 10 is connected through a capacitor CI to a terminal 14. The terminal 14 is connected through a resistor RI to a reference voltage source 15 and also through the collector emitter path of the transistor TI to the earth line. The base of transistor TI receives a series of pulses from a timing circuit 16. The components described so far are conventional in pacemaker circuits. In operation, the capacitor CI is charged by the reference voltage source 15 and, each time a pulse is supplied to the base of transistor TI, a stimulus pulse is supplied across the electrodes 11 and 13. Each stimulus pulse is variable both in amplitude and duration.

The reference voltage source 15 and terminal 14 are connected to the two inputs of an amplifier 17 which

subtracts the voltage appearing at terminal I4 from the reference voltage $V_{ref}$, amplifies the result, inverts it and adds to it an offset equal in magnitude to $V_{ref}$. The output of amplifier I7 is connected through an analog switch SI to a sample and hold circuit I9. The output of circuit I9 is connected to an amplifier 20 and analog switch S2 to terminal I4. The switches SI and S2 are controlled by the timing circuit I6.

In operation, immediately upon termination of each stimulus pulse, switch SI is closed and switch S2 is opened for a preset period and the output of amplifier I7 is sampled and held in circuit I9. Then, switch SI is opened and switch S2 is closed for a preset period and a voltage pulse is applied to terminal I4 having a magnitude and polarity which is determined by the output of sample and hold circuit I9. This pulse causes some of the charge which has been stored between electrodes II and I3 during the stimulus pulse to be removed. The cycle is then repeated a predetermined number of times thereby returning the voltage at terminal I4 to a value substantially equal to $V_{ref}$. Because there is no appreciable change in the voltage across capacitor CI, the series of operations returns the potential at the stimulating electrode substantially to that of the earth line. The intrinsic pulses may then be observed at terminal I4.

Thus, the intrinsic pulses are not masked by the charge which has been stored between electrodes II and I3

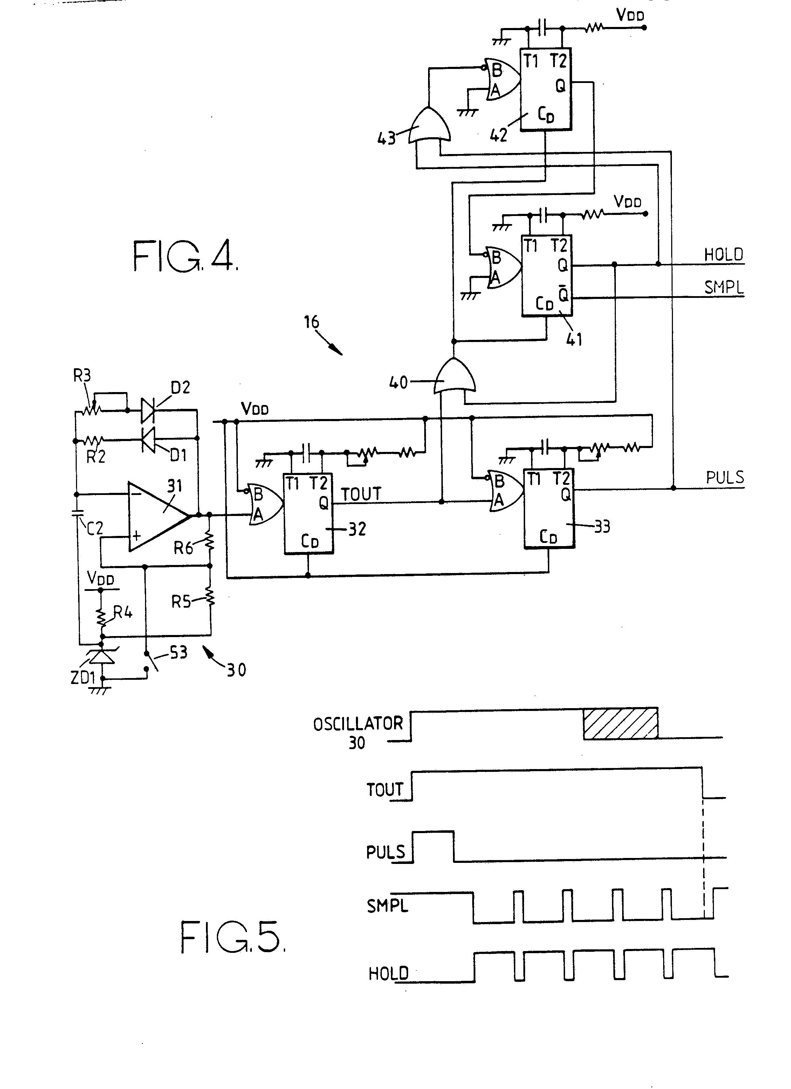

The detailed circuit diagram of the pacemaker circuit shown in Figure 2 will now be described with reference to Figures 3 to 5.

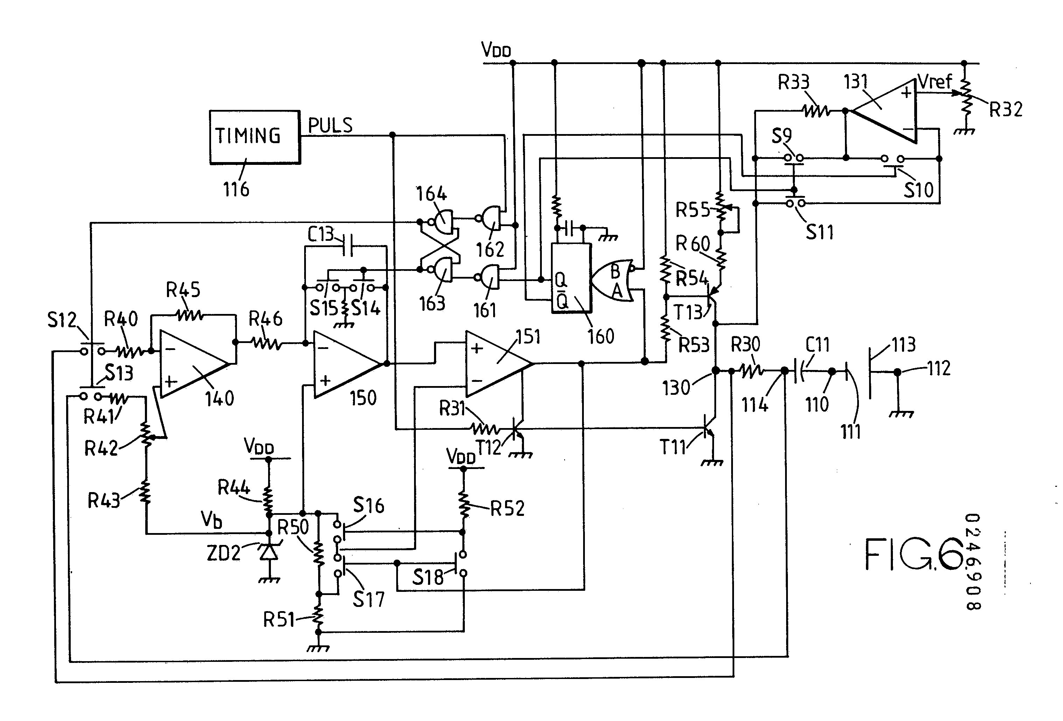

Referring firstly to Figure 4, the timing circuit I6 includes an oscillator 30. The oscillator comprises a type 3I40 operational amplifier 3I, the output of which is connected to its inverting-input through a diode DI and a resistor R2. The output is also connected to the inverting input through a diode D2 and a preset resistor R3. The inverting input is connected through a capacitor C2 to the junction of a Zener diode ZDI and a resistor R4 which are connected in series between the positive supply rail and the earth line to provide a bias voltage. This junction is also connected through a resistor R5 and a resistor R6 to the output of amplifier 3I and the junction of these two resistors is connected to the non-inverting input. The non-inverting input is also connected to the earth rail through a switch S3 which may be closed to prevent oscillation. The oscillator 30 provides the basic timing signal of the pacemaker circuit and the resistor R3 may be adjusted to control the frequency of the stimulus pulses. The output of amplifier 3I is connected to the A input of a type 4528 monostable 32 which produces a signal TOUT at its Q output. The Q output of this monostable is connected to the input of a further type 4528 monostable 33 at the Q output of which is produced a signal PULS. As shown in Figure 3, the signal PULS is connected to the base of transistor TI through a resistor R7. The monostable 33 is set to provide pulses having a variable duration.

The Q output of monostable 32 is connected to one input of an OR gate 40, the output of which is connected to the enable inputs $C_D$ of a pair of monostables 4I and 42. The monostable 4I is set to produce pulses having a duration of 0.4ms and monostable 42 is set to produce pulses having a duration of 25 microseconds. The Q output of monostable 4I is connected to one input of an OR gate 43, the output of which is connected to the B input of monostable 42. The Q output of monostable 42 is connected to the B input of monostable 4I. The Q output of monostable 4I is also connected to the other input of OR gate 40 and the Q output of monostable 33 is connected to the other input of OR gate 43. The Q output of monostable 4I produces a signal HOLD and is connected to the control terminal of switch S2. The $\overline{Q}$ output of monostable 4I produces a signal SMPL which is connected to the control terminal of switch SI.

A waveform diagram is shown in Figure 5 for the signal appearing at the output of oscillator 30 and for the signals TOUT, PULS, SMPL, and HOLD. As may be observed in Figure 5, the signal TOUT controls the number of cycles of operation of switches SI and S2. It may also be observed in Figure 5 that the switch SI is always closed immediately upon termination of each stimulation pulse.

Referring more particularly to Figure 3, the voltage reference source I5 comprises resistor RI0, a potentiometer RII and resistor RI2 connected in series between the positive supply rail $V_{DD}$ and the earth line. The tapping on potentiometer RII provides the reference voltage $V_{ref}$. This junction is connected to the non-inverting input of a type 3I40 operational amplifier 50.

The amplifier I7 comprises three type 3I40 operational amplifiers 5I, 52 and 53 which are connected as an instrumentation amplifier. The non-inverting input of amplifier 5I is connected to terminal I4 and the non-inverting input of amplifier 52 is connected to the tapping on potentiometer RII. The output of amplifier 5I is connected through a resistor RI3, a preset resistor RI4 and I resistor RI5 to the output of amplifier 52. The junction of resistors RI3 and RI4 is connected to the inverting input of amplifier 5I and the junction of resistors RI4 and RI5 is connected to the inverting input of amplifier 52. The output of amplifier 5I is connected through a resistor RI6 to the inverting input of amplifier 53. The output of amplifier 52 is connected through a resistor RI7, a potentiometer RI8, and a resistor RI9 to the output of amplifier 50 and the tapping on potentiometer RI8 is connected to the non-inverting input of amplifier 53. The output of amplifier 53 is connected to its inverting input through resistor R20.

The output of amplifier 53 represents the output of amplifier I7 and is connected through a resistor R2I and switch SI to a capacitor C4 which represents the sample and hold circuit I9. The capacitor C4 is a low leakage polyester type capacitor. It is to be noted that the time constant of resistor R2I in capacitor C4 is 2.5 microseconds which is one tenth of the duration of the pulses of the signal SMPL.

The amplifier 20 is formed from a type 3I40 operational amplifier 60 and a pair of transistors T3 and T4 connected between the positive supply line $V_{DD}$ and the negative supply line $V_{SS}$. The emitters of

transistors T3 and T4 are commonly connected to the inverting input of amplifier 60 to provide a voltage follower arrangement. The non-inverting input of amplifier 60 is connected to the junction of switch SI and capacitor C4 and the emitters of transistors T3 and T4 are connected through the switch S2 to terminal I4.

In order to minimise external interferene, the lines between the non-inverting input of amplifier 5I and terminal I4 and the non-inverting input of amplifier 52 and the tapping on potentiometer RII are screened and the entire circuit is enclosed in a die cast box which is earthed. It is also to be noted that the timing circuit I6 is arranged so that there are no logic level transitions during the sampling periods as such transitions could cause internal interference.

Referring now to Figure 6, there is shown the circuit diagram of another pacemaker circuit. As will be explained in more detail, in this pacemaker circuit the charge which is stored between the stimulating electrode and the myocardium during each stimulus pulse is measured by using an integrator and a measuring resistor and a substantially equal amount of charge is then removed upon termination of the stimulus pulse.

The circuit shown in Figure 6 has a stimulating electrode III and a reference electrode II3. The electrode II3 is connected to the earth line via a terminal II2. The stimulating electrode III is connected via a terminal II0 and a capacitor CII to a terminal II4. Terminal II4 is connected through a measuring resistor R30 to a terminal I30. The terminal I30 is connected to the earth line through the collector/emitter path of a transistor TII. The pacemaker circuit includes a timing circuit II6 which provides a pulsed signal PULS similar to the signal PULS produced by the circuit shown in Figure 4. The signal PULS is supplied through a resistor R3I to the base of transistor TII. The pacemaker circuit further includes a potentiometer R32 connected between the positive supply line $V_{DD}$ and the earth line. A tapping on potentiometer R32 provides a reference voltage $V_{ref}$ and this reference signal is supplied to the non-inverting input of a type 3I40 operational amplifier I3I. The output of amplifier I3I is connected through a resistor R33 to terminal I30. The output of amplifier I3I is also connected through an analog switch SI0 to its non-inverting input. Its non-inverting input is also connected through an analog switch SII to terminal I30 and an analog switch S9 is connected between the output of amplifier I3I and terminal I30. With the exception of switches S9, SI0 and SII and the measuring resistor R30, the components described so far are conventional in a pacemaker circuit. In operation, with the switch SI0 closed and switches S9 and SII open, amplifier I3I charges the capacitor CII to the voltage $V_{ref}$. Each time a pulse is supplied to the base of transistor TII, the stimulus pulse is applied between electrodes III and II3.

Terminal I30 is connected through an analog switch SI2 and a resistor R40 to the inverting input of a type 3I40 operational amplifier I40. The terminal II4 is connected through an analog switch SI3, a resistor R4I, a potentiometer R42, and a resistor R43 to the junction of a Zener diode ZD2 and a resistor R44 connected in series between the positive supply line and the earth line. The resistor R44 and Zener diode ZD2 operate to provide a bias voltage $V_b$. A tapping on potentiometer R42 is connected to the non-inverting input of amplifier I40 and its output is connected to its inverting input through a I megohm feed back resistor R45. Thus, operational amplifier I40 together with its associated components amplifies the voltage appearing across the measuring resistor R30.

The output of operational amplifier I40 is connected through a resistor R46 to the inverting input of a type 3I40 operational amplifier I50. The non-inverting input of amplifier I50 receives the bias voltage $V_b$. The output of amplifier I50 is connected through an integrating capacitor CI3 to its inverting input. The output of amplifier I50 is also connected to its non-inverting input through a pair of analog switches SI4 and SI5, the junction of switches SI4 and SI5 being connected through a resistor to the earth line. Thus, amplifier I50 can integrate the current flowing through resistor R30 and so operate as an integrator. When the switches SI4 and SI5 are closed the integrator is reset.

The output of amplifier I50 is connected to the positive input of a type 3II comparator I5I. The junction of resistor R44 and Zener diode ZD2 is connected through a resistor R50 and the resistor R5I to the earth line. Resistor R50 is bridged by a pair of analog switches SI6 and SI7 and the junction of these switches is connected to the negative input of comparator I5I. A resistor R52 and a further analog switch SI8 are connected between the positive supply line $V_{DD}$ and the earth line. The junction of resistor R52 and switch SI8 is connected to the control terminal of switch SI6. The control terminals of switches SI7 and SI8 are commonly connected together to the output of amplifier I5I. Also, the signal PULS is connected through resistor R3I to the base of a transistor TI2, the emitter of which is connected to the earth line and the collector of which is connected to the enable input (pin 6) of amplifier I5I.

The output of comparator I5I is connected through a resistor R53 and the resistor R54 to the positive supply rail $V_{DD}$ and the junction of these two resistors is connected to the base of a transistor TI3. The emitter of transistor TI3 is connected through a resistor R54 and a potiometer R55 to the positive supply line $V_{DD}$ and the collector of this transistor is connected to terminal I30. Thus, when transistor TI3 is on, this transistor and the associated resistors operate as a current source.

The output of amplifier I5I is connected to the A input of a type 4528 monostable I60. The Q output of this monostable is connected to the control terminal of switch SII and the $\overline{Q}$ output is connected to the control terminal of switch SI0. The Q output of monostable I60 is also connected to one input of a NAND gate I6I. NAND gate I6I is associated with another NAND gate I62, one input of which receives the signal PULS. The other input of NAND gates I6I and I62 are connected to the positive supply line $V_{DD}$. The output of NAND gate I6I is connected to one input of a NAND gate I63 and the output of NAND gate I62 is connected to one input of a NAND gate l64. The NAND gates l63 and l64 are connected as a R-S flip flop. The output of NAND gate l63 is connected to the control terminals of switches Sl3 and Sl4 and the output of NAND gate l64 is connected to the control terminals of switches Sl2 and Sl3.

In operation, immediately before the leading edge of each pulse of the signal PULS, switches Sl2 and Sl3 are open, switches Sl4 and Sl5 are closed. Consequently, the output of amplifier l50 is clamped to the bias voltage $V_b$. At the leading edge of the pulse of the signal PULS, transistors Tll and Tl2 are turned on, thereby supplying a stimulus pulse across electrodes lll and ll3 and disabling comparator l5l. The leading edge of the pulse also causes the output of NAND gate l64 to go high, thereby closing switches Sl2 and Sl3 and opening switches Sl4 and Sl5. Consequently, during the stimulus pulse, the current flowing through resistor R30 is integrated by amplifier l50 and the deviation of the output of this amplifier between the beginning and the end of the pulse represents the charge stored between electrodes lll and ll3. Upon termination of the stimulus pulse, transistors Tll and Tl2 turn off and comparator l5l is enabled. As the output of amplifier l50 is now below the bias voltage $V_b$, the output of comparator l5l goes low thereby turning on transistor Tl3 which acts as a current source for removing the charge which has been stored. During charge removal, the current flowing through resistor R30 is again integrated by comparator l50. When the output of comparator l50 reaches the bias voltage $V_b$, the output of comparator l5l goes high thereby turning off transistor Tl3, triggering monostable l60, closing switches Sl7 and Sl8 and opening switch Sl6. The operation of switches Sl6 to Sl8 provides hysteresis for the negative input of comparator l5l. As a result of triggering monostable l60, the output of NAND gate l63 goes high and so switches Sl4 and Sl5 are closed thereby removing the charge on capacitor Cl3 and switches Sl2 and Sl3 are opened. Whilst the Q output of monostable l60 is high, switches S9 and Sll are closed and switch Sl0 is open. This has the effect of clamping terminal l30 to the reference voltage $V_{ref}$ thereby substantially removing any remaining charge stored between electrodes lll and ll3. When the Q output of monostable l60 goes low, switches S9 and Sll open and switch Sl0 closes and the reference voltage $V_{ref}$ is then applied to terminal l30 via a resistor R33.

Therefore, upon termination of each stimulation pulse, the charge stored between electrodes lll and ll3 is substantially removed by transistor Tl3. In practice, it has been found that the charge is removed at this stage to an accuracy of approximately 0.l%. Because the interface between the stimulus electrode lll and the myocardium has complex non-linear properties, it is unlikely that this interface will be returned precisely to its initial state and it is for this reason that terminal l30 is then clamped for a preset period to the reference voltage $V_{ref}$. When the terminal l30 is supplied with a reference voltage via resistor R33, the intrinsic pulses in the heart may be sensed at terminal l30.

Thus, in the two embodiments of the invention which have been described, the charge which is stored between the stimulus electrode and the myocardium during the stimulus pulse is subsequently removed thereby making it possible to detect the evoked heart pulses. The evoked pulses may therefore be used for diagnosis whilst the pacemaker circuit is operating. For example, damaged tissue in a heart may cause a secondary wave to follow the due PRST waves and this wave may be detected using the present invention. Also, the pacemaker circuit of the present invention may be modified so that it can detect a specific component of the evoked pulse and use this to modify the operation of the pacemaker circuit. For example, the detection of such a pulse may be used to modify the frequency or intensity of the stimulus pulses or to suppress or trigger them as may be appropriate.

## Claims

l. A cardiac pacemaker circuit comprising a terminal (l0) for connection to a stimulating electrode (ll), a terminal (l2) for connection to a reference electrode (l3), means (l5, Tl) for applying a stimulus pulse across said terminals in response to a timing signal, means for generating said timing signal (l6), and means (l7, l9) operable on termination of said stimulus pulse to reduce charge stored between said electrodes whereby for enabling monitoring of intrinsic pulses.

2. A pacemaker as claimed in claim l wherein said stimulus pulse applying means comprises a reference voltage source (l5) coupled to said stimulus terminal (l0) and switch means (Tl) responsive to said timing signal to apply said stimulus pulse from said reference voltage source to said stimulus terminal.

3. A pacemaker as claimed in claim 2 wherein said reference voltage source (l5) is coupled through a series resistance capacitance circuit (Rl, Cl) to said stimulating terminal (l0) and said switch means (Tl) is connected between the junction of said resistance and capacitance and said reference terminal (l2).

4. A pacemaker as claimed in claim 2 or 3 wherein said charge reducing means (l7, l9) is operable to sample the voltage difference between said stimulus terminal (l0) and said reference voltage on termination of said stimulus pulse and to reduce said stored charge in dependence on said voltage difference.

5. A pacemaker as claimed in claim 4 wherein said charge reducing means (l7, l9) is operable repeatedly to sample said voltage difference and reduce said stored charge after each said sample until the voltage across said stimulus and reference terminals (l0, l2) is reduced to a value substantially equal to zero.

6. A pacemaker as claimed in any preceding claim wherein said charge removing means (l7, l9) is operable to apply a pulse of preselected duration across said stimulus and reference

terminals (I0, I2) of a polarity to reduce said stored charge.

7. A pacemaker circuit as claimed in claim 4 or 5, or claim 6 when apendent to claim 4 or 5 wherein said charge removing means (I7, I9) comprises a comparator (I7) for comparing said reference voltage with said stimulus terminal voltage and generating an output signal in dependence on said comparison; a sample and hold means (I9) for sampling said comparator output signal and applying a signal to said stimulus terminal to reduce said stored charge in dependence on the level of said output signal; and control means for controlling operation of said sample and hold means.

8. A pacemaker as claimed in claim I or 2 wherein said charge reducing means (I7, I9) is operable to monitor the quantity of charge supplied to said stimulus terminal (I0) by a stimulus pulse and to remove substantially an identical quantity of charge on termination of the stimulus pulse.

9. A pacemaker as claimed in claim 8 wherein said charge reducing means (I7, I9) is operable to clamp said stimulus electrode (I0) at said reference voltage level for a preset period after removal of said substantially identical quantity of charge.

I0 A pacemaker as claimed in claim 8 or 9 further comprising a resistance (R30) connected in series with one of said terminals (II0,II2) and wherein the charge supplied during said stimulus pulse and the charge removed are measured by integrating the voltage across said resistance (R30) with respect to time.

FIG.1(a)

0246908

FIG.1(b)

FIG.2.

FIG.3.

0246908

# FIG.4.

HOLD
SMPL

16

PULS

# FIG.5.

OSCILLATOR
30

TOUT

PULS

SMPL

HOLD

FIG.4.

FIG.5.

FIG.6

0246908